# EUROPEAN PATENT APPLICATION

(11) **EP 1 243 928 A1**
(43) Date of publication of application: **25.09.2002**
(21) Application number: 00987709.3
(22) Date of filing: 22.12.2000
(51) Int. Cl.: G01N 33/86, G01N 33/48

(54) **MEANS OF STABILIZING COMPOSITIONS AND REAGENTS**

(30) Priority: 24.12.1999 JP 36666399
(71) Applicant: INTERNATIONAL REAGENTS CORPORATION, Kobe-shi Hyogo 651-0083 (JP)
(72) Inventor: OKUDA, Masahiro, Int. Reagents Corp. Res. Dev. Ct, Kobe-shi, Hyogo 651-2241 (JP); HIURA, Hisahide, Int. Reagents Corp. Dev. Ctr., Kobe-shi, Hyogo 651-2241 (JP)
(74) Representative: Forstmeyer, Dietmar, Dr. rer. nat., Dipl.-Chem.
(86) International application number: JP0009131
(87) International publication number: WO01048486

(57) **Abstract**

A means for stable preparing a composition, which comprises plasma component derived from human or animals, at least fibrinogen, which is blood coagulation factor, and factor V, is provided, and the composition prepared remains stable even when allowed to stand over one day or longer in a freeze-dried state, in freeze-thawed state, in a refrigerator, or under room temperature. By adding an inhibitor of Factor XIII in an effective amount for achieving the inhibitory effect, compositions comprising human or animal plasma components, at least fibrinogen and Factor V, which are blood coagulation factors, and reagents for measuring extrinsic coagulation factor for blood coagulation function comprising at least thromboplastin, Factor V and fibrinogen can be stabilized during preparation, storage and measurement.

## Description

### Field of the Invention

Present invention is related to compositions containing blood plasma for use in measuring blood coagulation function and regents for measuring extrinsic coagulation factor for blood coagulation function in clinical examinations.

More particularly, the present invention is related to a means for stabilizing these compositions and reagents, and is further related to compositions and reagents introduced in accordance with said means for stabilization.

### Background of the Invention

Compositions comprising human or animal plasma components, at least fibrinogen and Factor V, which are blood coagulation factor, likely form insoluble precipitate, when allowed to stand over 1 day or more under the conditions, such as in freeze-drying, freeze-thawing state, and in a refrigerator or under room temperature. Accordingly, a production and preparation of the stable compositions comprising these components have been considered as being difficult so far.

Regents for measuring extrinsic coagulation factor for blood coagulation function are incorporated with thromboplastin, Factor V and fibrinogen, which are derived from human or animal (Acta Med. Scad., suppl., 194, 1947). Since such reagents likely cause insoluble precipitate when allowed to stand over 1 day or more, especially under the condition, such as in freeze-drying, freeze-thawing state, and in refrigerator or under room temperature, the reagent was difficult to produce and prepare in stable condition. Beside these, when reagents are used for measuring blood coagulation function, problem is also caused that stable results could not be obtained, due to a stability thereof, prepared on demand.

### Detailed Description of the Invention

An object of the present invention is to provide a means for preparing stable compositions, comprising human or animal blood plasma components, at least fibrinogen and Factor V, which are one of blood coagulation factor.

Another object of the present invention is to provide stable compositions prepared under the stabilizing condition, and even when allowed to stand over 1 day or more in freeze-dried state, in a freeze-thawing state, and in a refrigerator or under room temperature.

Further object of the present invention is to provide the composition, which shows excellent stability after preparing thereof on demand.

As a result of culminated search works by the present inventors, the inhibitor of Factor XIII had been found to effectively stabilize the compositions comprising human or animal blood plasma components, especially comprising at least fibrinogen and Factor V, which are blood coagulation factor. Thus, the present invention was attained successfully.

That is, according to the present invention,
(1) Means for stabilizing compositions, which are derived from plasma comprising at least fibrinogen and Factor V, characterized by inhibiting the biological function of Factor XIII,
(2) Means for stabilizing reagents for measuring extrinsic coagulation factor for blood coagulation function, comprising at least fibrinogen, Factor V and thromboplastin or lipidated tissue factor, characterized by inhibiting biological function of Factor XIII,
(3) Means for stabilizing compositions derived from blood plasma comprising at least fibrinogen and Factor V, characterized by adding the inhibitor of Factor XIII, in an effective amount for inhibiting thereof,
(4) Means for stabilizing reagents, for measuring extrinsic coagulation factor for blood coagulation function, comprising at least fibrinogen, Factor V and thromboplastin or lipidated tissue factor, characterized by adding the inhibitor for Factor XIII, in an effective amount for inhibition reagents
(5) Compositions containing blood plasma which is introduced with the said stabilizing means (1) or (3), and
(6) Reagents for measuring extrinsic coagulation factor for blood coagulation function which is introduced in accordance with said stabilizing means (2) or (4).

### Best Mode for reduction practice of the Invention

The present invention is described in more detailed as follows.

Thromboplastin used in the present invention, is provided by conventionally extracting from human placenta, and brains of such as bovine, monkey or rabbit, and the like. In place of thromboplastin, a lipidated tissue factor, prepared by recombination of tissue factor apoenzym lipidated with suitable phospholipids may be used.

On the other hand, as for Factor V and fibrinogen, purely improved commercially available products may be used. However, for example, plasma, obtained from bovine plasma by absorbing at least Factor VII, Factor X, and Factor II, with barium sulfate, etc., has been conventionally used.

The reagent for measuring extrinsic coagulation factor for blood coagulation function is prepared by mixing above mentioned thromboplastin or lipidated tissue factor, with plasma containing at least Factor V, and fibrinogen, which are removed according to the above-mentioned absorbing step.

The reagent, thus prepared, may be prepared conventionally by optional adding with, if desired, suitable buffer solution, antiseptic, and calcium ion, which may also be blood coagulation factor.

If the composition, thus prepared, is allowed to stand still for one or more days, under freeze-dried state, a freeze-thawing state, and a refrigerator or under room temperature, an insoluble matter is produced. Thus, the stability of the composition becomes significantly worse.

According to the present invention, it has been found that, the stabilization for the composition, derived from plasma, which contains at least fibrinogen and Factor V, and the reagent for measuring complex blood coagulation factor, which contains at least fibrinogen and Factor V and thromboplastin, or lipidated tissue factor, may be made by adding effective inhibiting amount of inhibitor for Factor XIII.

Herein below, the means for stabilizing the composition, derived from plasma, or the reagent for measuring extrinsic coagulation factor for blood coagulation function, are explained with referring to the inhibitor for Factor XIII. The stabilizing method according to the present invention is, however, attained by using a matter, which is capable of inhibiting biological function of Factor XIII, and the present invention is not restricted to the inhibitor for Factor XIII. Factor XIII, discussed herein, means functions for stabilizing fibrin by forming cross-linked bonding thereto with transglutaminase function, and the like.

The inhibiting agent for Factor XIII is exemplified below. The most preferable is maleimide derivatives, which are exemplified as concrete examples.

### (Examples of the inhibiting agent)

Maleimide derivatives:
N-ethyl maleimide, N- (4-dimrthylamino-3, 5-dinitrophenyl) maleimide, N, N'-hexamethylene bis-maleimide, N, N'-para-phenylene dimaleimide, hexamethylene bismaleimide bis (N-maleimide methyl) ether, etc.

Alkyl halide and derivatives thereof:
monochloroacetic acid and amid thereof, monoiode acetic acid and amide thereof, monobromo acetic acid and amide thereof, α-iode propionic acid, β-bromoethylamine, mono-chloro acetic acid, chloro acetifenone, and etc.

Disulfide compound:
5,5'-dithiobis(2-nitro benzoic acid), dithio pyridine, and etc.

Others:
acryronitrile, acrylamide, ethylacrylate, acrolein, maleic anhydride, vinylsulfone, ethyleneimine, ethylene oxide, p-chloromercury(II)benzoicacid,
p-chloromercury(II)denylsulfonate, glycine, glycylglycine, glycylmethyl ester, cadaverine, glysin amide, and etc.

These inhibitors maybe used alone or two or more in combination. Addition amount of the inhibitor may be appropriately decided in consideration of an amount of Factor XIII, to be added, as well as an effective amount of each inhibitor. As for density, in the case of liquid solution of N-ethylmaleimide, prior to freeze drying step, 0.1 mM to 10 mM are suitable, and more preferably 1mM to 2mM. However, the addition amount may be properly decided by repeated with simple experiments, so that a density may not precipitate insoluble matter. The ranges mentioned above, are not always limited.

### Example

The present invention is described in more detailed with referring to the following examples, to which are not limited. Unless otherwise specified, parts, and percents are by weight.

### Example 1

### (Preparation of thromboplastin)

As a raw material for preparing thromboplastin, a fresh or freeze-thawed brain of bovine, rabbit or monkey, were used. Blood coagulation factors contaminated was sufficiently removed by water washing. The brain tissue, which was prepared by homogenizing in Warring Blender with adding acetone to form acetone powder with no fat, or which had been homogenized in Warring Blender with physiological saline, and followed by repeatedly several centrifuge and homogenization treatments, were used. Said brain tissue was suspended in the physiological saline, and was extracted in vibrating extraction apparatus at 48°C for 30 minutes, and separated under centrifuge to obtain supernatant as thromboplastin solution. This method was according to the process described in the literature (Zoku Kagaku Jikken Koza 8, Under blood stream, 497p, published by Tokyo Kagaku Dojin).

In alternate method, the lipidated tissue factor was prepared by recombinant tissue factor lipidated with proper phospholipids. The recombinant lipidated tissue factor was obtained by mixing 0.2 µg of recombinant rabbit or bovine tissue factor, and 0.2 mg of liposome consisting of phosphatidyl choline and phosphatidyl serine (mixing ratio: 7:3), previously prepared, in 2ml of Hepes-Tris buffer solution (pH 7.0)containing 0.2 % deoxycholic acid, was incubated at 37°C for 1 hour and was dialyzed against 1 litter of 20mM Hepes-Tris buffer solution(pH 7.0) for 24 hours, to obtain recombinant tissue factor. This method was according to a method described in the literature (Method Enzymol. 222, p.177, 1993.

### Example 2

### (Preparation of plasma derived composition/ absorpted plasma, containing at least fibrinogen and Factor V)

To 100 ml of bovine plasma, barium sulfate in 20 w/v % was added. The mixture was mixed for 30 minutes at room temperature, and stirred slowly. Subsequently, the mixture was centrifuged at 5,000xg to remove the barium sulfate precipitate, and absorbed plasma was prepared by adding in the supernatant, and was mixed with 1 ml of N-ethylmaleimide, as inhibitor for Factor XIII. In other words, the composition prepared, contained at least Factor V and fibrinogen. Beside these, the absorbed plasma, without containing 1 mL of N-ethylmaleimide, was prepared and used, as a control.

### Example 3

### (Preparation of reagent for measuring extrinsic coagulation factor for blood coagulation function)

To one volume of thromboplastin derived from freeze-thawed bovine brain, obtained in the Example 1, together with one volume of the absorbed plasma, obtained in the Example 2, one volume of a solution, which was prepared by adding 10 mM calcium lactate to 20 mM Hepes-Tris buffer solution (pH 7.0), was formed as a reagent for measuring extrinsic coagulation factor for blood coagulation function. A reagent for measuring extrinsic coagulation factor for blood coagulation function, without containing 1mM of N-ethylmaleimide, prepared in the Example 2, was prepared.

### Example 4

### (Effect of various kind of inhibitor)

The absorbed plasma was prepared in the same manner as described in the Example 2, except that N-ethylmaleimide was not contained. Separately, one volume of the absorbed plasma and one volume of 20 mM Hepes-Tris buffer solution (pH 7.0), in which 10 mM of calcium lactate was added, were mixed together to prepare the solution. To the solution, each of substance, stated in Table 1 as below, was added so that mixture might show such a concentration as mentioned in the Table 1. With these mixtures, the inhibiting rate for factor XIII, and a producing rate of the insoluble matter after storage for 24 hours at 10°C were measured. The results are shown in the Table 1 as below. As clearly seen in the Table, the prohibition for insoluble precipitation may be clearly attained by inhibiting factor XIII using the substances, which are capable of reacting with thiol groups.

**Table 1**

| **Inhibiting effect for insoluble precipitation using Various substances** | | | |
|---|---|---|---|
| | Concentration | Inhibiting for factor XIII | Precipitation |
| N-ethylmaleimide | 1 mM | + | - |
| N-(4-dimethylamino-3,5-dinitrophenyl) maleimide | 1 mM | + | - |
| Monoiodo acetic acid | 1 mM | + | - |
| Bovine serum albumin | 1% | - | + |
| Sucrose | 5% | - | + |
| NaCl | 10 mM | - | + |
| Inhibiting for XIII factor + :Yes - :No | | | |
| Precipitation + :Yes - :No | | | |

### Example 5

### (Concentration adjustment of the inhibitors)

The absorbed plasma was prepared in the same manner as stated in the Example 2, except that a concentration of N-ethylmaleimide was varied. Separately, one volume of the absorbed plasma and one volume of 20 mM Hepes-Tris buffer solution (pH 7.0), in which 10 mM of calcium lactate was added, were mixed together to prepare the solution, for which a producing rate of the insoluble matter was visually detected both after storing 24 hours at 10 °C, and right after freeze-thawed at -20°C. The results are shown in Table 2 as below. As clearly seen in the Table 2, it has been cleared that N-ethylmaleimide may inhibit insoluble precipitation even with adding 1 mM concentration, however, more preferably 1 mM to 10 mM.

**Table 2**

| **Influence of N-ethylmaleimide concentration for Inhibiting effect for insoluble precipitation** | | | | |
|---|---|---|---|---|
| Concentration (mM) | Bovine plasma 1 | | Bovine plasma 2 | |
| | 10°C | -20°C | 10°C | -20°C |
| 0 | + | ++ | + | ++ |
| 0.1 | ± | + | ± | + |
| 1 | - | - | - | - |
| 2 | - | - | - | - |
| 5 | - | - | - | - |
| 10 | - | - | - | - |

| | | | | |
|---|---|---|---|---|
| + : Insoluble Precipitation | | | | |
| ± : Slightly insoluble precipitation | | | | |
| - : No insoluble precipitate | | | | |

### Example 6

### (Storage effect by adding inhibiting agent for plasma derived composition/adsorbed plasma, containing at least fibrinogen and Factor V)

To study the effect of the present invention, one volume of three types absorbed plasma, prepared in the Example 2 and one volume of 20 mM Hepes-Tris buffer solution were mixed together, and visually detected both after storing 24 hours at 10 °C, and right after freeze-thawed at 20°C. The results are shown in Table 3 below. As clearly seen in the Table 3, none of the adsorbed plasma mixed with N-ethylmaleimide observed the insoluble precipitation, neither in storing at 10°C or after freeze-thawed treatment. On the contrary, all those without adding N-ethylmaleimide showed insoluble precipitation both in storing at 10°C and after freeze-thawed treatment. Thus, it has become clear that the insoluble precipitation can be inhibited by adding N-ethylmaleimide.

**Table 3**

| **Adding effect of N-ethylmaleimide in three example of the adsorbed plasma** | | | | |
|---|---|---|---|---|
| Plasma | Storage at 10 °C | | Storage after freeze-thawing at -20 °C | |
| | NEM(-) | NEM(+) | NEM(-) | NEM(+) |
| Adsorbed plasma 1 | + | - | ++ | - |
| Adsorbed plasma 2 | + | - | ++ | - |
| Adsorbed plasma 3 | + | - | ++ | - |

| | | | | |
|---|---|---|---|---|
| + : Insoluble precipitation | | | | |
| - : No insoluble precipitation | | | | |
| NEM : N-ethylmaleimide | | | | |

### Example 7

### (Stabilizing effect in agent for measuring extrinsic coagulation factor for blood coagulation function)

In order to further study the effect of the present invention, reagents for measuring extrinsic coagulation factor for blood coagulation function were prepared in the same manner, as shown in Example 4, using the absorbed plasma, except that the concentrations of the N-ethylmaleimide were changed to 1 mM, 1.5 mM, and 2 mM, respectively. The effects were confirmed, as shown in Table 4.

The reagent, without using N-ethylmaleimide, recognized insoluble precipitation after one day, and contrarily, none of the reagents containing 1 mM, 1.5 mM, and 2 mM, recognized insoluble precipitate at all. Thus, even in the reagents for measuring extrinsic coagulation factor for blood coagulation function, it has been found that the insoluble precipitation was inhibited by adding N-ethylmaleimide, and stability of the reagent was successively confirmed.

**Table 4**

| **Adding effect for N-ethylmaleimide (NEM)** (Presence of the insoluble precipitate in the reagents for measuring extrinsic coagulation factor for blood coagulation function) | | | | | | |
|---|---|---|---|---|---|---|
| Agents | Additives (mM NEM) | 0 day | 1day | 2days | 3days | 4days |
| Prior reagent | 0 | - | + | + | + | ++ |
| Reagent of the invention | 1 | - | - | - | - | - |
| Reagent of the invention | 1.5 | - | - | - | - | - |
| Reagent of the invention | 2 | - | - | - | - | - |

| | | | | | | |
|---|---|---|---|---|---|---|
| +: Insoluble precipitation | | | | | | |
| -: No insoluble precipitation | | | | | | |
| NEM : N-ethylmaleimide | | | | | | |

### Example 8

### (Effect of the inhibiting reagent affected to measure performance)

In order to confirm the performance of each reagent, prepared in the Example 8, the performance was evaluated using Blood coagulation analyzer, Coagre (manufactured by Shimazu, Co., Japan) by measuring clotting time with commercially available control plasma. The performance and stability of the reagent evaluated by using the reagents, prepared and preserved for several days at 10°C, to obtain plasma coagulation activity of control plasma. The results are shown in Table 5. As clearly seen in the Table 5, clotting time was not varied even adding NEM.

The reagent without adding NEM showed decreasing in the blood coagulation activity with duration of time from 1 to 4 days, and the reagent containing 1 mM, 1.5 mM, or 2 mM of NEM showed no decreasing in the blood coagulation activity at all. Thus, it has been found that the addition of the NEM is able to inhibit lowering reagent activity and to stabilize the reagent on the performance of the reagent for measuring extrinsic coagulation factor for blood coagulation function.

**Table 5**

| **Adding effect of NEM affected on the property of the reagent for measuring coagulation activity** (Unit: Activity %) | | | | | | |
|---|---|---|---|---|---|---|
| Agents | Additives (mM NEM) | 0 day | 1day | 2days | 3days | 4days |
| Prior agent | 0 | 100 | 95 | 90 | 88 | 82 |
| Agent of the invention | 1 | 100 | 102 | 104 | 104 | 99 |
| Agent of the invention | 1.5 | 100 | 101 | 103 | 103 | 98 |
| Agent of the invention | 2 | 100 | 100 | 101 | 103 | 101 |

### Example 9

### (Effect of other inhibitors)

The procedures stated in the Examples 5 to 8 were repeatedly carried out using the mixtures of NEM with monochloroamide, acrylamide, glycinamide, and dithioprydine, as an inhibiting agent, in an amount of 1 : 1, as other representative examples. These mixtures showed the equal or slightly inferior to NEM, and stabilizing effect of the present invention was surely confirmed.

### Possible availability in commercial industry

As explained in detailed above, the present invention is to provide a means for stabilizing a composition, which comprises plasma components derived from human or animals, at least fibrinogen and Factor V, which are blood coagulation factor. The means are attained by adding effective amount for attaining inhibiting effect of the inhibitor for Factor XIII. By introducing said means, an reagent for measuring extrinsic coagulation factor for blood coagulation function, which is stable in preparing, storing, and measuring steps, is provided. A reagent for measuring complex blood coagulation property, containing above components, and at least thromboplastin, Factor V and fibrinogen, is provided.

## Claims

1. A means for stabilizing a composition, which is derived from plasma, comprising at least fibrinogen and Factor V, **characterized by** inhibiting biological function for Factor XIII.

2. A means for stabilizing an agent, which is for measuring a extrinsic coagulation factor for blood coagulation function, comprising at least fibrinogen, Factor V, and thromboplastin or lipidated tissue factor, **characterized by** inhibiting biological function for Factor XIII.

3. The means for stabilizing the composition according to claim 1, wherein an inhibitor for Factor XIII is added in an effective amount.

4. The means for stabilizing the agent according to claim 2, wherein an inhibitor is added in an amount for attaining inhibiting effect.

5. A composition, which is derived from plasma, comprising at least fibrinogen and Factor V, **characterized by** inhibiting biological function for Factor XIII, or **characterized by** being stabilized by adding effective amount of an inhibitor for Factor XIII.

6. An agent, which is for measuring a extrinsic coagulation factor for blood coagulation function, comprising at least fibrinogen, Factor V, and thromboplastin or lipidated tissue factor, **characterized by** inhibiting biological function for Factor XIII, or **characterized by** being stabilized by adding effective amount of an inhibitor for Factor XIII.
